# EUROPEAN PATENT SPECIFICATION

(11) **EP 0 901 777 B1**
(45) Date of publication and mention of the grant of the patent: **07.05.2003**
(21) Application number: 98306497.3
(22) Date of filing: 14.08.1998
(51) Int. Cl.: A61F 2/34

(54) **Prosthesis assembly**
Prothesenanordnung
Ensemble prothétique

(30) Priority: 15.08.1997 GB 9717391
(43) Date of publication of application: 17.03.1999
(73) Proprietor: Johnson & Johnson Medical Limited, Ascot, Berkshire SL5 9EY (GB)
(72) Inventor: Hiernard, Bruno Georges Eugene, Bransgore, Christchurch, Dorset BH23 8BJ (GB); Bigsby, Robert John Andrew, Lymington, Hampshire SO41 0QX (GB)
(74) Representative: Fisher, Adrian John

(56) References cited:
- EP-A- 0 585 503
- WO-A-85/00284
- FR-A- 2 651 995
- FR-A- 2 677 878
- FR-A- 2 682 588

## Description

This invention relates to prosthesis assembly, in particular, a shell for insertion into the acetabulum of a patient and an insert for providing a bearing surface for a hip joint.

Hip joint prostheses are known in which a shell component is attached to the acetabulum of a patient by means of cement or directly to the bone with or without screw fixation. An insert generally in the form of a cup is positioned within the shell. The inner surface of the cup provides a bearing surface for the femoral head.

European patent no. EP0436317B discloses an insert which has an extended bearing surface by extending the rim of the insert upwardly away from the cup form. The insert disclosed in this document is formed of a metal or polyethylene material. WO 8 500 284, FR 2 682 588, FR 2 677 878 and FR 2 651 995 also disclose inserts having an enlarged rim.

It is preferable to form a cup shaped insert of a ceramic material which has better wear characteristics and biocompatibility than other materials but is very brittle due to its low fracture toughness. The brittle characteristics of ceramic materials mean that cup shaped inserts formed of ceramic materials cannot have an extended bearing surface as any area of the cup not supported by the shell is liable to fracture.

According to the present invention there is provided a prosthesis assembly comprising: a fixation element for attachment to the bone of the patient; a ceramic bearing cup component with a rim; wherein a portion of the rim is augmented, the augmentation being in the form of a shoulder extending generally radially from the cup component providing an increased width of a portion of the rim. The ceramic material may be Alumina or Zirconia.

Preferably, the fixation element is formed of a synthetic plastics material or metal.

Preferably, there is a taper fit between the fixation element and the bearing cup component. The bearing cup component may have an outer surface which is tapered to form a male portion. The fixation element may have an internal surface which is tapered to form a female portion, corresponding to the male portion of the bearing cup component.

Preferably, the rim of the bearing cup component has a substantially oval shape.

Preferably, the internal bearing surface of the cup component is off-centre within the oval rim of the cup component.

Preferably, the prosthesis assembly is an acetabulum cup assembly.

An embodiment of a prosthesis assembly in accordance with the present invention will now be described with reference to the accompanying drawings in which:
Figure 1 is a cross-section through the prosthesis assembly;
Figure 2 is a perspective view of the prosthesis assembly;
Figure 3 is a plan view of the bearing cup component of the prosthesis assembly; and
Figure 4 is a perspective of the bearing cup component of the prosthesis assembly.

Referring to the drawings, there is provided a prosthesis assembly 1 formed of a fixation element in the form of a shell 2 and a bearing cup component in the form of an insert 4.

The shell 2 has a stepped external surface 6 which is attached to the bone of a patient. In this example, the prosthesis assembly 1 is an acetabula cup assembly for a hip replacement and the shell 2 will be attached to the acetabulum of the patient.

The shell 2 is attached to the acetabulum by means of a press fit which may have the addition of screw fixings. The screw fixings are inserted through bore holes 24 in the shell 2. The heads of the screw fixings are sunk into the shell 2 to avoid interference with the insert 4. Alternatively, the shell 2 can be cemented to the acetabulum of the patient.

The shell 2 is generally hemispherical in shape and has a flat rim 26. The shell 2 has a tapered portion 14 adjacent the rim 26 of the shell 2.

The insert 4 is in the form of a cup with an inner surface 12 which is the bearing surface for the joint component in this example, the femoral head. The outer surface 28 of the insert 4 has a tapered portion 16 which provides a male taper which fits within the female taper 14 of the shell 2. The remaining portion of the outer surface 28 of the insert 4 is dimensioned such that it does not bear on the inner surface of the shell 6. The space between the outer surface 28 of the insert 4 and the inner surface 22 of the shell 2 is required for the taper 6 between the shell 2 and the insert 4 to operate.

The insert 4 has a rim 8 which has an augmented portion 10 which it extends radially from the cup shape of the insert 4. The augmented portion of the rim 10 forms a shoulder extending outwardly over the flat rim 26 of the shell 2. A gap 18 is provided between the flat rim 26 of the shell 2 and the augmented portion of the rim 10.

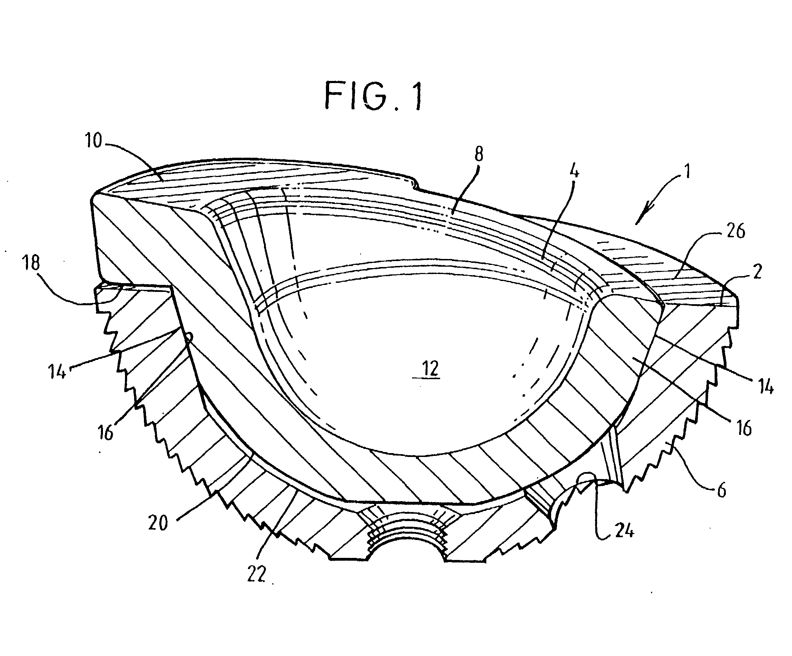

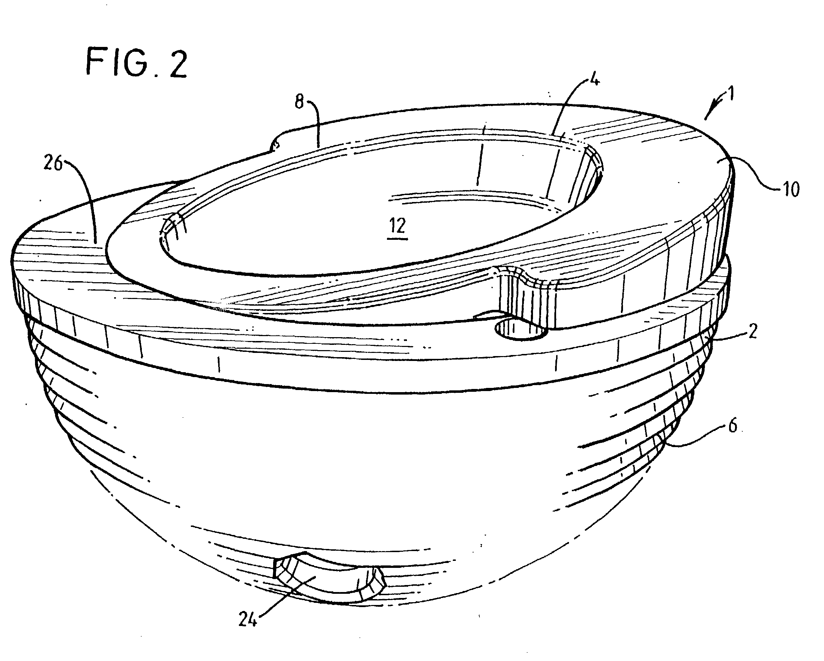

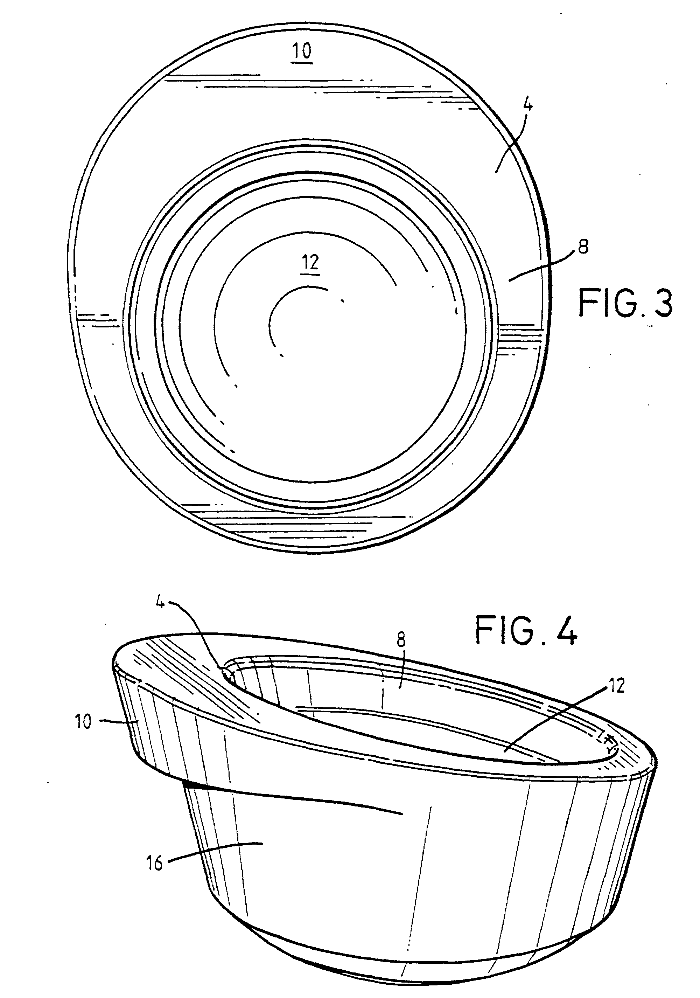

As can been seen from Figure 3, the rim 8 of the insert 4 has an oval shape with the hemispherical bearing surface 12 being off-set from the centre of the oval shape. As shown in Figures 1 and 2, the rim 8 of the insert 4 may extend more abruptly into the augmented portion of the rim 10.

Two ceramic materials are currently available for orthopaedic devices, namely zirconia and alumina.

Alumina is a much harder material than zirconia and for the best result it is recommended to use the same material for the femoral head and the bearing insert. It is, however, possible to replace a ceramic head with a cobalt-chromium head. The alumina/alumina articulation has the best characteristics but the material is very brittle. It is known that the cobalt-chromium/polyethylene articulation has a wear rate between 0.2 and 0.5 mm per year. An alumina/alumina articulation has a wear rate between 0.0001 and 0.005 mm per year.

An augmented insert 4 is required as it is often difficult to attach the shell to the acetabulum in its exact position. This may be due to the anatomy of the patient, etc. It is therefore necessary to use an insert 4 with an offset bearing surface 12 which overcomes the problem of inexact positioning of the shell 2. The range of motion of the joint can be increased and the risk of dislocation can be reduced.

Ceramic inserts are known and there is virtually no risk of breaking insert if it is flush with the surface of the shell. If the bearing surface of the insert is augmented, a portion of the rim is unsupported and thus the risk of breaking the ceramic insert is very high.

When the surgeon operates, he first fixes the shell into the acetabulum and then endeavours to find the optimum position for augmented insert 4 by using a trial insert 4 made from a biocompatible plastic. Once the surgeon has determined the exact location required for the insert 4 he pushes the ceramic insert 4 into the shell 2. The insert 4 locks within the shell 2 by the co-operation of the tapered portions 14 and 16 on the insert 4 and the shell 2. A taper locking mechanism offers the possibility of rotating the insert 360 degrees inside the shell 2 thus giving complete positioning freedom. The surgeon then tries the range of motion and may slightly modify the position of the augmented insert 4 in the shell 2. Once satisfied, the surgeon impacts the insert 4 in the shell 2 to lock the two tapered components 14, 16 firmly together.

The present invention modifies the strength of the augmented insert 4 by increasing the rim thickness 10 at the highest unsupported portion of the rim 8. The increased strength of the augmented insert 4 allows the augmented insert to be formed of a ceramic material such as alumina or zirconia.

There must be a small gap 20 between the augmented portion 10 of the rim and the flat rim 26 of the shell 2 to allow the taper lock to operate.

Finite-element-analysis has been carried out on an augmented insert made of ceramic material in accordance with the prior art and on the proposed augmented insert 4. The results have shown that the stress is reduced with the augmented insert 4 of the present invention thus reducing the overall risk of breaking the insert 4.

Modifications and improvements can be made to the foregoing without departing from the scope of the present invention.

## Claims

1. A prosthesis assembly (1) comprising: a fixation element (2) for attachment to the bone of the patient; a bearing cup component (4) with a rim (8); wherein a portion (10) of the rim (8) is augmented, the augmentation (10) being in the form of a shoulder extending generally radially from the cup component (4) providing an increased width of a portion of the rim (8), **characterised in that**
the cup component (4) is formed of a ceramic material.

2. A prosthesis assembly (1) as claimed in claim 1, wherein the ceramic material is Alumina or Zirconia.

3. A prosthesis assembly (1) as claimed in either of the preceding claims, wherein the fixation element (2) is formed of a synthetic plastics material or metal.

4. A prosthesis assembly (1) as claimed in any of the preceding claims, wherein there is a taper fit between the fixation element (2) and the bearing cup component (4).

5. A prosthesis assembly (1) as claimed in claim 4, wherein the bearing cup component (4) has an outer surface (28) which is tapered to form a male portion (16).

6. A prosthesis assembly (1) as claimed in claim 5, wherein the fixation element (2) has an internal surface which is tapered to form a female portion (14), corresponding to the male portion (16) of the bearing cup component (4).

7. A prosthesis assembly (1) as claimed in any of the preceding claims, wherein the rim (8) of the bearing cup component (4) has a substantially oval shape.

8. A prosthesis assembly (1) as claimed in claim 7, wherein the internal bearing surface (12) of the cup component (4) is off-centre within the oval shape of the cup component (4).

9. A prosthesis assembly (1) as claimed in any of the preceding claims, wherein the prosthesis assembly (1) is an acetabulum cup assembly.

## Patentansprüche

1. Prothesenbaugruppe (1) umfassend: ein Befestigungselement (2) zur Anbringung am Knochen des Patienten; ein Lagerschalen-Bauteil (4) mit einem Rand (8), wobei ein Teil (10) des Randes (8) verstärkt ist und die Verstärkung (10) die Form einer Schulter hat, die sich allgemein in radialer Richtung vom Lagerschalen-Bauteil (4) aus erstreckt, wodurch ein Teil des Randes (8) mit größerer Breite entsteht, **dadurch gekennzeichnet, daß** das Lagerschalen-Bauteil (4) aus einem keramischen Material geformt ist.

2. Prothesenbaugruppe (1) nach Anspruch 1, bei welcher das keramische Material Aluminiumoxid oder Zirkoniumoxid ist.

3. Prothesenbaugruppe (1) nach einem der bisherigen Ansprüche, bei welcher das Befestigungselement (2) aus synthetischem Kunststoffmaterial oder aus Metall geformt ist.

4. Prothesenbaugruppe (1) nach einem der bisherigen Ansprüche, bei welcher es zwischen dem Befestigungselement (2) und dem Lagerschalen-Bauteil (4) eine Kegelpassung gibt.

5. Prothesenbaugruppe (1) nach Anspruch 4, bei welcher das Lagerschalen-Bauteil (4) eine Kegel-Außenfläche (28) aufweist, welche einen Außenkegel (16) bildet.

6. Prothesenbaugruppe (1) nach Anspruch 5, bei welcher das Befestigungselement (2) eine Kegel-Innenfläche aufweist, welche einen Innenkegel (14) bildet, der dem Außenkegel (16) des Lagerschalen-Bauteiles (4) entspricht.

7. Prothesenbaugruppe (1) nach einem der bisherigen Ansprüche, bei welcher der Rand des Lagerschalen-Bauteiles (4) eine im wesentlichen ovale Form hat.

8. Prothesenbaugruppe (1) nach Anspruch 7, bei welcher die innere Lagerfläche (12) des Lagerschalen-Bauteiles (4) exzentrisch innerhalb der ovalen Form desselben liegt.

9. Prothesenbaugruppe (1) nach einem der bisherigen Ansprüche, welche eine Hüftgelenkpfannen-Baugruppe ist.

## Revendications

1. Ensemble prothétique (1) comprenant un élément de fixation (2) pour la fixation à l'os du patient ; un composant de cuvette de roulement (4) avec une couronne (8) ; dans lequel une partie (10) de la couronne (8) est augmentée, l'augmentation (10) se présentant sous la forme d'un épaulement qui s'étend généralement de façon radiale à partir du composant de cuvette (4) procurant une largeur augmentée d'une partie de la couronne (8), **caractérisé en ce que** le composant de cuvette (4) est formé avec un matériau en céramique.

2. Ensemble prothétique (1) selon la revendication 1, dans lequel le matériau en céramique est de l'alumine ou de la zircone.

3. Ensemble prothétique (1) selon l'une des revendications précédentes, dans lequel l'élément de fixation (2) est formé avec une matière plastique synthétique ou du métal.

4. Ensemble prothétique (1) selon l'une quelconque des revendications précédentes, dans lequel il existe un montage conique entre l'élément de fixation (2) et le composant de cuvette de roulement (4).

5. Ensemble prothétique (1) selon la revendication 4, dans lequel le composant de cuvette de roulement (4) présente une surface externe (28) qui est conique pour former une partie mâle (16).

6. Ensemble prothétique (1) selon la revendication 5, dans lequel l'élément de fixation (2) présente une surface interne qui est conique pour former une partie femelle (14), correspondant à la partie mâle (16) du composant de cuvette de roulement (4).

7. Ensemble prothétique (1) selon l'une quelconque des revendications précédentes, dans lequel la couronne (8) du composant de cuvette de roulement (4) a une forme sensiblement ovale.

8. Ensemble prothétique (1) selon la revendication 7, dans lequel la surface de roulement interne (12) du composant de cuvette (4) est excentrée à l'intérieur de la forme ovale du composant de cuvette (4).

9. Ensemble prothétique (1) selon l'une quelconque des revendications précédentes, dans lequel l'ensemble prothétique (1) est un ensemble de cuvette de cavité cotyloïde.
